Europäisches Patentamt

European Patent Office

Office européen des brevets

(11) Veröffentlichungsnummer: **0 255 881 B1**

## (12) EUROPÄISCHE PATENTSCHRIFT

(45) Veröffentlichungstag der Patentschrift: **18.09.91**

(51) Int. Cl.⁵: **A61F 5/01, A61F 13/10**

(21) Anmeldenummer: **87110112.7**

(22) Anmeldetag: **14.07.87**

(54) **Bandage für die Behandlung von Unterarmbeschwerden.**

(30) Priorität: **31.07.86 DE 3625983**

(43) Veröffentlichungstag der Anmeldung:
**17.02.88 Patentblatt 88/07**

(45) Bekanntmachung des Hinweises auf die
Patenterteilung:
**18.09.91 Patentblatt 91/38**

(84) Benannte Vertragsstaaten:
**AT BE CH DE ES FR GB IT LI NL SE**

(56) Entgegenhaltungen:
**DE-A- 319 343**
**US-A- 3 785 371**

(73) Patentinhaber: **Röder, Georg**
**Harksheider Strasse 157**
**W-2000 Hamburg 65(DE)**

(72) Erfinder: **Röder, Georg**
**Harksheider Strasse 157**
**W-2000 Hamburg 65(DE)**

(74) Vertreter: **Glaeser, Joachim, Dipl.-Ing. et al**
**Patentanwalt Königstrasse 28**
**W-2000 Hamburg 50(DE)**

## Beschreibung

Es ist bekannt, daß Beschwerden und Schmerzen im Unterarm, die auf Überbeanspruchungen vor allem durch häufige Muskelanspannungen der Unterarme (daher der Name Tennisarm) oder durch monotone Arbeiten in ungünstiger Lage zurückzuführen sind, durch eine eng am Unterarm anliegende Bandage, die auf die Sehnen einen Druck in Richtung auf die Speiche bzw. die Elle ausübt, so daß Beschwerden insbesondere durch Epicondylitis bzw. Epicondylopathie während des Tragens der Bandage gemildert werden.

Nachteilig bei derartigen bekannten Bandagen (US-A-3 785 371) ist, daß sich ihre Wirkung nur dann einstellt, wenn sie einen ausreichend hohen Druck ausüben, d. h. so eng anliegen, daß sie zwangsläufig die Blutzirkulation beeinträchtigen, sie dürfen daher nicht dauernd sondern nur kurze Zeit während der Arbeit oder beim Sport getragen werden, so daß sie ihre therapeutische Wirkung kaum entfalten können.

Die vorliegende Erfindung betrifft Bandagen, die diese Nachteile vermeiden, also monate- oder jahrelang ohne Unterbrechung getragen werden können und,wie die Praxis bereits gezeigt hat, nach wenigen Monaten Tragens eine vollständige, mindestens langfristig anhaltende Ausheilung der Sehnenscheide sowie der Sehnenansätze erbringen.

Dies wird im wesentlichen durch die besondere Formgebung der Bandage nach Anspruch 1 erzielt, welche bewirkt, daß die Bandage das Ellbogengelenk und die anschließenden Teile des Ober-und Unterarmes sowohl im Ruhezustand wie auch bei Bewegungen, welche die Epicondylen nicht wesentlich belasten, nur knapp aber nicht eng umschließt, sich aber vor allem an den kritischen Stellen der Sehnen bei merklicher Beanspruchung derselben so eng anlegt, daß die Zugrichtung der Sehnen der Unterarmmuskulatur flacher, d. h. näher tangential zur Speiche bzw. zur Elle verläuft als dies ohne die Bandage der Fall wäre und einen Konterzug in Richtung der Epicondylen ausübt.

Der Zuggurt stellt einen Entlastungsschluß für die Sehnenenden dar, weil er die erforderliche Zugkraft von dem vorderen Teil des Kragens, dort wo der Kragen den kleineren Durchmesser hat, bis zum Oberarm führt. Eine einstückige Ausbildung dieser beiden Teile ist dann möglich, wenn die Kraftangriffspunkte für die Zugentlastung vor denjenigen Bereichen liegen, an denen die Druckkräfte auf die Sehnen ausgeübt werden. Bevorzugt wird eine Ausführungsform der Erfindung, in der die beiden Funktionen von einer einstückigen Bandage ausgeübt werden, die aus ebenem Material gestanzt werden kann.

Ein weiteres Merkmal der erfindungsgemäßen Bandage kann darin bestehen, daß sie zwei über ein Verbindungsstück miteinander einstückig verbundene gürtel- oder kragenartige Teile enthält, welche die dem Ellbogen benachbarten Teile des Ober- und Unterarmes umschließen und zwischen denen eine den Unterteil des Ellbogens freilassende Ausnehmung vorgesehen ist.

Obwohl die erfindungsgemäße Bandage demnach estens zwei Funktionen ausübt, besteht sie gemäß einer bevorzugten Ausgestaltung aus einem einzigen entsprechend gestalteten Stück eines geeigneten biegsamen Materials, vorzugsweise Leder oder Kunstleder, das gegebenenfalls auch mit einem weicheren Material wie Stoff, Filz o. dgl. gefüttert ist, und auf dem nur einzelne Applikationen wie Schnallen, Klettverschlüsse u. dgl. nachträglich angebracht sind. Diese bevorzugte Ausführungsform bietet nicht nur den fertigungstechnischen Vorteil, daß die Bandage bzw. ihr weitaus größter und wichtigster Teil in einem einzigen Arbeitsgang gestanzt werden kann, sondern verbürgt darüber hinaus ein besonders gutes Zusammenwirken ihrer einzelnen Teile.

Vorzugsweise wird sowohl der den Oberarm umschliessende Zuggurt als auch der den Unterarm umschließende kragenartige Teil von Riemen gebildet, die um den Ober- bzw. den Unterarm geschlungen und miteinander mittels geeigneter verstellbarer Verbindungsmittel wie Klettverschlüssen oder Gürtelschließen mit der für den Träger optimalen Weite verbunden werden.

Die Bandage gemäß der Erfindung bewirkt, daß bei Belastung des Armes der Kragen in eine Position gebracht wird, die eine optimale Entlastung und Ruhigstellung der Sehnenansätze sowohl des äußeren wie auch des inneren Epicondylus, der Sehnenscheide und gleichzeitig auch des Ellbogengelenkes und einen Teil des Oberarmes bewirken. Bei Kraftanstrengungen stellt die Bandage dadurch einen Entlastungsschluß für die Sehnenansätze her, während sie bei nicht belastetem Arm locker am Arm anliegt, wobei die Bandage gemäß der Erfindung die Bewegungsfreiheit des Armes in keiner Weise beeinträchtigt, d.h. der Arm kann in seine gestreckte Lage gebracht werden.

Es könnte auch eine Bandange gemäß der Erfindung zweiteilig ausgebildet werden, indem der vordere Teil für sich für die Zugentlastung sorgt, während der dahinter liegende Teil für die Ausübung der Druckkraft auf die Sehnen herangezogen wird.

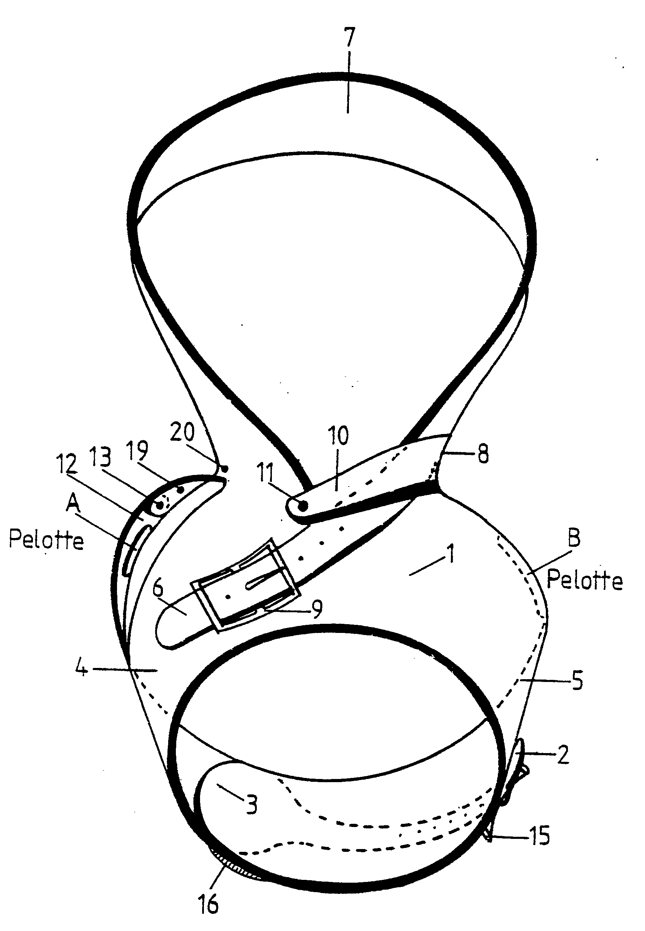

Die Erfindung wird nachstehend an Hand der Zeichnung beispielsweise erläutert, welche eine schaubildliche Darstellung einer Bandage für den rechten Arm zeigt.

In der Figur bedeuten 2 und 3 die Endstücke von zwei Riemenendabschnitten 4 und 5, die einen Kragen 1 bilden, welcher den Unterarm einer Person umschließen kann. Der Kragen 1 wird dadurch angelegt, daß der Riemenendabschnitt 4 über den bereits um den Unterarm geschlungenen Riemenendabschnitt 5 gelegt und mit Hilfe von Befestigungsmitteln 15 (im dargestellten Beispiel eine Gürtelschließe mit Dorn) in einer Lage fixiert wird, in der der gemäß der Erfindung konisch ausgebildete Kragen 1 genau auf den kritischen Stellen A und B (dort wo sich Pelotten befinden können) des Unterarms druckausübend aufliegt.

Am oberen Teil des Kragens 1 ist ein Gurtansatz 7 ausgebildet, der sich von dorther bei Blickrichtung der Figur in die Tiefe der Zeichnung erstreckt und um das untere Ende des Oberarmes gelegt werden kann und über einen an ihm befestigten freien Endstück 6 mit dem vorderen Teil des Kragens über den Laschenansatz 8 mit einer Verbindung 9 einstellbar befestigt werden kann.

Die Teile 7, 8, 6 und 9 stellen die wesentlichen Teile der vorliegenden Erfindung dar, es gibt keine bekannten Manschetten, die ähnliche oder entsprechende Teile aufweisen. In jedem Falle sind diese Teile so ausgebildet, daß sie eine Zugkraft (bei Betrachtung der Zeichnung von vorne her nach hinten in die Tiefe der Zeichnung) ausüben können, so daß der Kragen 1 bzw. dessen Teile 3, 4 und 5 bei Beanspruchung der Muskulatur fest auf die inneren und äußeren Muskelstränge des Unterarms gezogen werden.

Vorteilhafterweise wird der Gurtansatz 7 bzw. dessen freies Endstück 6 auf dem Laschenansatz 8 mit Hilfe einer an diesem oder dem oberen Teil des Kragens 1 befestigten Lasche 10 fixiert, die über den Gurtansatz 7 gelegt und in dieser Lage durch einen Druckknopf 11 festgelegt werden kann. Somit ist gewährleistet, daß der Zuggurt bei Belastung nicht über den Ellbogen rutscht.

Ein weiteres wichtiges bevorzugtes Merkmal einer Ausführungsform der Erfindung stellt eine vom oberen Teil des Kragens 1 abzweigende Lasche 12 dar, deren freies Ende mit dem Unterteil des Gurtansatzes 7 oder an einem Vorsprung des Gurtansatzes 7 in gewünschter Lage verbunden ist. Die Lasche 12 kann mit Hilfe einer Niet oder eines Druckknopfs 13 in gezeigter Lage oder in Öffnungen 19 und 20 des Gurtansatzes 7 verbunden werden. Wesentlich ist dabei, daß die Lasche 12 eine Länge hat, die kürzer ist als zum Erreichen des Gurtansatzes 7 in ebenem Zustand der ausgebreiteten Bandage nötig wäre.

Durch diese Maßnahme wird ein Druck auf die äußeren Sehnenpartien des Unterarmes ausgeübt, sobald und solange diese eine durch Muskelkontraktion verursachte Dickenzunahme aufweisen.

Gemäß einer weiteren Ausgestaltung der Erfindung ist an der Außenseite des Endstückes 3 des unteren Riemenendabschnittes 5 ein Aufsatz 16 angebracht, z.B. aufgesteppt oder aufgeklebt, auf den sich der andere untere Riemenendabschnitt 4 aufstützen kann und damit die dauerhaft gut anliegende Paßform verbürgt. Diese Ausführungsform bietet, besonders in Verbindung mit der bereits geschilderten Art des Anlegens (erst Bildung des Kragens) den Vorteil, daß der einmal gebildete gut sitzende Kragen 1 und der Zuggurt 7 nie mehr geöffnet zu werden brauchen, sondern auch biem Ablegen und Wiederanlegen erhalten bleiben.

Zwecks Abstreifens bzw. Wiederanlegens des Kragens 1 wird dessen Öffnung durch Abheben des Riemenendabschnittes 4 vom Aufsatz 16 erweitert. Das Auf- oder Abstreifen des Zuggurtes 7 wird ebenfalls ohne Öffnung desselben dadurch ermöglicht bzw. erleichtert, daß nach Lösen des Druckknopfes 11 die Lasche 10 den Zuggurt 7 freilegt und dieser über den Ellbogen geschoben werden kann.

**Patentansprüche**

1. Aus biegsamem Material bestehende Bandage für die Behandlung von durch Überbeanspruchung der Sehnenansätze und/oder der Sehnenscheide des Unterarms bedingten Beschwerden, aufweisend einen um den Unterarm legbaren Kragen (1), der aus einem Riemen gebildet wird, dessen zwei Endabschnitte (4,5) durch verstellbare Verbindungsmittel (15) miteinander verbunden werden, dadurch gekennzeichnet, daß der Kragen (1) außen Befestigungsmittel aufweist und innen vorzugsweise mit mindestens einer Pelotte ausgestattet ist und daß sich vom oberen Teil des Kragens (1) ein um den Oberarm legbarer Zuggurt (7) erstreckt, dessen freies Ende (6) mit Hilfe von verstellbaren Befestigungsmitteln (9) mit dem oberen Teil des Kragens (1) verbindbar ist.

2. Bandage nach Anspruch 1, gekennzeichnet durch eine vom oberen Rand des einen Riemenendabschnittes (4) des Kragens (1) abzweigende, zum oberen Gurtansatz (7) reichende Lasche (12), die nahe zu ihrem Endpunkt mit dem oberen Gurtansatz (7) verbindbar ist.

3. Bandage nach Anspruch 2, dadurch gekennzeichnet, daß die Länge der Lasche (12) kürzer bemessen ist als zum Erreichen des oberen Gurtansatzes (7) in völlig eben ausgebreitetem Zustand der Bandage erforderlich.

4. Bandage nach den Ansprüchen 2 und 3, dadurch gekennzeichnet, daß zur Verbindung des Endes der Lasche (12) mit dem oberen Gurtansatz (7) ein Niet oder ein Druckknopf (13) dient.

5. Bandage nach den Ansprüchen 1 bis 4, dadurch gekennzeichnet, daß mindestens eines der verstellbaren Befestigungs- oder Verbindungsmittel (15 oder 9) ein Klettverschluß ist.

6. Bandage nach den Ansprüchen 1 bis 5, dadurch gekennzeichnet, daß mindestens eines der verstellbaren Verbindungsmittel (15 oder 9) eine Gürtelschließe mit Dorn ist.

7. Bandage nach den Ansprüchen 1 bis 6, gekennzeichnet durch einen auf der Außenseite des Endstückes (3) eines der unteren Riemenendabschnitte (5) angebrachten Aufsatz (16).

8. Bandage nach den Ansprüchen 1 bis 7, gekennzeichnet durch eine auf einem oberen Laschenansatz (8) angebrachte oder ausgestanzte Lasche (10) und einem Mittel (11) zur Befestigung ihres freien Endes am Gurtansatz (7).

9. Bandage nach Anspruch 8, dadurch gekennzeichnet, daß das Befestigungsmittel (11) ein Druckknopf ist.

10. Bandage nach den Ansprüchen 1 bis 9, dadurch gekennzeichnet, daß sie bis auf nachträglich aufgebrachte Applikationen wie Klettverschlüsse, Gürtelschnallen u. dgl. aus einem einzigen Stück biegsamen Materials besteht.

11. Bandage nach den Ansprüchen 1 bis 10, dadurch gekennzeichnet, daß sie im wesentlichen aus - gegebenenfalls gefüttertem - Leder, Gewebematerial, Vlies oder Kunststoff besteht.

## Claims

1. Bandage made of flexible material for the treatment of complaints caused by an overstrain of the tendon attachment or the tendon sheath of the forearm, comprising a collar (1) to be put around the forearm, which collar is formed by a belt, the two end sections (4, 5) of which are connected to one another by adjustable connection means (15) characterized in that at its outside the collar (1) comprises attachment means and at its inside it is provided preferably with at least one pressure pad, and that from the upper part of the collar (1) there extends a pulling strap (7) to be put around the upper arm, the free end (6) of the pulling strap being adapted to be connected with the upper part of the collar (1) by means of adjustable attachment means (9).

2. Bandage according to claim 1, characterized by a tab (12) branching off from the upper edge of the one belt end section (4) of the collar (1) and extending to the upper strap attachment (7), which tab (12) close to its end can be connected with the upper strap attachment (7).

3. Bandage according to claim 2, characterized in that the length of the tap (12) is shorter than necessary for reaching the upper strap attachment (7) in a completely plane spreaded state of the bandage.

4. Bandage according to claims 2 and 3, characterized in that for connecting the end of the tap (12) with the upper strap attachment (7) a rivet or a press button (13) is used.

5. Bandage according to claims 1 to 4, characterized in that at least one of the adjustable attachment means or connections means (15 or 9) is a velcro strip.

6. Bandage according to claims 1 to 5, characterized in that at least one of the adjustable connection means (15 or 9) is a belt buckle with tongue.

7. Bandage according to claims 1 to 6, characterized by a fixture (16) mounted to the outside of the end piece (3) of one of the lower belt end sections (5).

8. Bandage according to claims 1 to 7, characterized by a tap (10) disposed at or punched out from the upper tap attachment (8) and a means (11) for adjusting its free end to the strap attachment (7).

9. Bandage according to claim 8, characterized in that the attachment means (11) is a press button.

10. Bandage according to claims 1 to 9, characterized in that it consists of one single piece of flexible material except for subsequently applied attachments like velcro strips, belt buckles and the like.

11. Bandage according to claims 1 to 10, characterized in that it substantially consists of - optionally lined - leather, textile fabric, fleece or plastic material.

## Revendications

1. Bandage en matière flexible pour le traitement de fatigues et douleurs limitées de l'avant-bras, par mise sous surtension des attaches ou gaines des tendons, comprenant une manchette qui peut être fixée autour de l'avant-bras et qui est formée d'une bande dont les deux parties d'extrémité (4, 5) peuvent être reliées entre elles par des moyens de liaison réglables (15), caractérisé en ce que la manchette (1) porte des moyens de fixation à l'extérieur et est pourvue d'au moins une pelotte à l'intérieur et en ce qu'une sangle de traction (7), qui peut être fixée autour du bras s'étend à partir de la partie supérieure de la manchette (1), l'extrémité libre de cette sangle pouvant être reliée à la partie supérieure de la manchette à l'aide de moyens de fixation réglables (9).

2. Bandage selon la revendication 1, caractérisé par une languette (12) qui se détache du bord supérieur d'une des parties d'extrémité (4) de la manchette (1), en allant jusqu'à l'extension qui forme la sangle supérieure (7), et peut être reliée à celle-ci près de son extrémité.

3. Bandage selon la revendication 2, caractérisé en ce que la longueur de la languette (12) est plus faible que celle qui est nécessaire pour atteindre l'extension qui forme la sangle supérieure (7) quand le bandage est complètement étalé à plat.

4. Bandage selon l'une des revendications 2 ou 3, caractérisé en ce qu'un rivet ou un bouton-pression (13) sert à relier l'extrémité de la languette (12) à l'extension qui forme la sangle supérieure (7).

5. Bandage selon l'une des revendications 1 à 4, caractérisé en ce qu'au moins un des moyens de fixation ou de liaison réglables (15 ou 9) est une liaison à auto-accrochage.

6. Bandage selon l'une des revendications 1 à 5, caractérisé en ce qu'au moins un des moyens de liaison réglables (15 ou 9) est du type boucle de ceinture à ardillon.

7. Bandage selon l'une des revendications 1 à 6, caractérisé par une garniture (16) placée sur la face extérieure de la partie terminale (3) d'une des parties d'extrémité inférieures (5) de la manchette.

8. Bandage selon l'une des revendications 1 à 7, caractérisé par une languette (10) montée sur une extension porte-languette supérieure (8), ou obtenue par découpage à la presse, et un moyen (11) pour fixer son extrémité libre de cette languette sur l'extension qui forme la sangle (7).

9. Bandage selon la revendication 8, caractérisé en ce que le moyen de fixation (11) est un bouton-pression.

10. Bandage selon l'une des revendications 1 à 9, caractérisé en ce qu'il est formé d'une seule pièce de matière flexible, à l'exception des accessoires rapportés ensuite tels que liaison à auto-accrochage, boucle de ceinture et analogue.

11. Bandage selon l'une des revendications 1 à 10, caractérisé en ce qu'il est constitué essentiellement de cuir, tissu, feutre ou matière synthétique, éventuellement pourvu d'un revêtement.